Europäisches Patentamt

European Patent Office

Office européen des brevets

(19)

(11) Veröffentlichungsnummer: **0 049 767**

**A2**

(12) ## EUROPÄISCHE PATENTANMELDUNG

(21) Anmeldenummer: 81107261.0

(22) Anmeldetag: 15.09.81

(51) Int. Cl.³: **G 02 C 13/00**
G 02 C 7/04, A 45 C 11/04
A 61 L 2/18

(30) Priorität: 14.10.80 DE 3038800

(43) Veröffentlichungstag der Anmeldung:
21.04.82 Patentblatt 82/16

(84) Benannte Vertragsstaaten:
CH DE FR GB IT LI

(71) Anmelder: Titmus Eurocon Kontaktlinsen GmbH & Co.
KG
Goldbacher Strasse 57
D-8750 Aschaffenburg(DE)

(72) Erfinder: Ludwig, Gerhard, Dr.
Strombergerstrasse 24
D-6531 Weiler(DE)

(74) Vertreter: Liedl, Gerhard et al,
Patentanwälte Liedl, Nöth, Zeitler Steinsdorfstrasse 21 -
22
D-8000 München 22(DE)

(54) Anordnung für die Pflege und/oder Aufbewahrung von Kontaktlinsen.

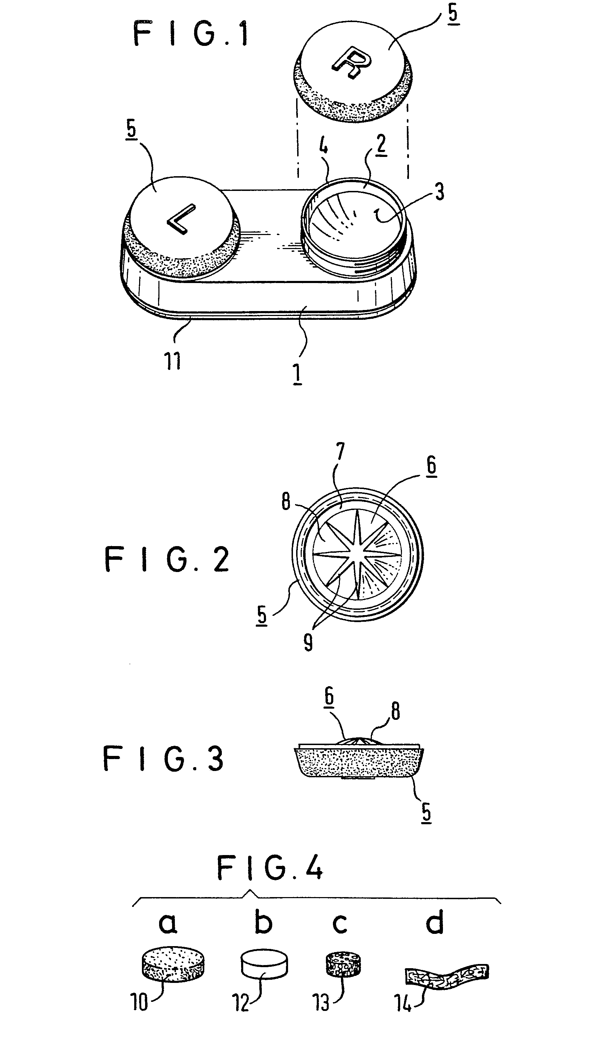

(57) Eine Anordnung zur Pflege und/oder Aufbewahrung von Kontaktlinsen mit einem Behälter 2, in dessen Innern die Kontaktlinse in eine Pflege- und/oder Aufbewahrungslösung eingelegt wird, wobei mit der Pflege- und/oder Aufbewahrungslösung eine ein antimikrobiell (oligodynamisch) wirksames Schwermetall in die Pflege- und/oder Aufbewahrungslösung abgebende Einheit 3 in Berührung steht (Fig. 1).

FIG.1

EP 0 049 767 A2

Anordnung für die Pflege und/oder Aufbewahrung von Kontaktlinsen

Die Erfindung betrifft eine Anordnung für die Pflege und/oder Aufbewahrung von Kontaktlinsen (weiche oder harte Kontaktlinsen) mit einem Behälter, in dessen Innern die Kontaktlinse in eine Pflege- und/oder Aufbewahrungslösung eingelegt wird.

Für die verschiedenen auf dem Markt befindlichen Kontaktlinsenmaterialien werden als geeignet spezifische Pflegesysteme, insbesondere Konservierungsmittel enthaltende Aufbewahrungslösungen, empfohlen. Dies führt z. B. in Familien, in denen Kontaktlinsen verschiedener Materialien getragen werden, dazu, daß mehrere unterschiedliche Pflegesysteme nebeneinander verwendet werden, d. h. pro Zeiteinheit wird eine relativ geringe Menge des einzelnen Präparats verbraucht, wodurch erhöhte Risiken hinsichtlich ausreichender Anbruchstabilität speziell in mikrobiologischer Sicht entstehen können. Außerdem werden an die Pflege- und Aufbewahrungslösungen hohe medizinische Anforderungen gestellt, die bei der Formulierung der Lösung sich in aller Regel nur mit Kompromissen erfüllen lassen. Dies gilt insbesondere für das in der Pflege- bzw. Aufbewahrungslösung vorhandene Konservierungsmittel, das dem Präparat nicht nur eine ausreichende Konservierungspotenz vermitteln soll, sondern auch gegen massive mikrobielle Rekontamination bei unsachgemäßer bzw. unsauberer Handhabung schützen soll. Hierbei soll eine ausreichend schnelle Sterilisationskinetik gewährleistet sein. Für die Pflege von harten Kontaktlinsen ist als konservierende Substanz in vielen Pflege- bzw. Aufbewahrungslösungen Benzalkoniumchlorid bekannt. Dieses birgt bei einer Konzentration über 0, 001 % die Gefahr schädigender Einwirkung auf das Endothel und die Hornhaut in sich. Außerdem wirkt diese Substanz erst über

BAD ORIGINAL

0,01 % sicher mikrobizid. Auch die Desinfektionswirkung gegenüber Pseudomonasaeruginosa ist mangelhaft. Darüber hinaus besteht bei Anwendung für weiche Kontaktlinsen die Gefahr einer stärkeren Anreicherung. Häufig wird zur Formulierung der Pflege- bzw. Aufbewahrungslösung auch Chlorhexidindigluconat verwendet, das bei Zutritt von Luftsauerstoff vermutlich durch intramolekulare Zyklisierung zu Verfärbungen neigen kann, die im Extremfall irreversibel auf die Kontaktlinsen übergehen können.

Das bedeutet, daß auch hier vorbeugende Maßnahmen bei der Formulierung der Lösung getroffen werden müssen.

Die Pflege und Aufbewahrung der Kontaktlinsen erfolgt in hierfür speziell ausgebildeten Behältern, wie eingangs schon erwähnt.

Aufgabe der Erfindung ist es, eine Anordnung der eingangs genannten Art zu schaffen, durch deren Ausbildung ohne großen Aufwand zumindest zum größten Teil auf ein Konservierungsmittel verzichtet werden kann und dennoch eine mikrobielle Kontamination auch nach mehrmaligem Öffnen des Behälters ausgeschlossen ist.

Diese Aufgabe wird erfindungsgemäß dadurch gelöst, daß mit der Pflege- und/oder Aufbewahrungslösung eine ein antimikrobiell (oligodynamisch) wirksames Schwermetall in die Pflege- und/oder Aufbewahrungslösung abgebende Einheit in Berührung steht.

Auf diese Weise läßt sich ein Aufbewahrungssystem für harte und weiche Kontaktlinsen schaffen, in welchem diese Kontaktlinsen in Pflegeund/oder Aufbewahrungslösungen aufbewahrt werden können, die nur einen geringen Gehalt an Konservierungsmittel, z. B. 25 % des üblichen Gehaltes, aufweisen und bei denen damit Nebenwirkungen der bekannten Konservierungsmittel vermieden werden können. Dieses Pfle-

gesystem eignet sich insbesondere für solche Personen, die chemische Pflegeverfahren nicht vertragen. Die Kontaktlinsen können im Behälter in einer unkonservierten Aufbewahrungslösung, insbesondere in einer sterilen isotonischen Kochsalzlösung, aufbewahrt bzw. gepflegt werden. Die Behälter können trocken aufbewahrt werden, so daß kein Keimwachstum möglich ist, wodurch eine Ersparnis von Aufbewahrungslösung sich erzielen läßt.

Die Einheiten, welche zur Abgabe des antimikrobiell (oligodynamisch) wirksamen Schwermetalls in die Pflege- und/oder Aufbewahrungslösung dienen, können äußerst einfach ausgebildet sein. Es kann sich hier beispielsweise um einen Preßling aus einem entsprechenden Schwermetallsalz handeln. Der Preßling kann Tablettenform oder eine sonstwie geeignete geometrische Gestaltung besitzen. Auch kann es sich um einen mit elementarem Schwermetall überzogenen Formkörper (Tablettenform) handeln. Geeignet sind auch poröse oder sonstige saugfähige Körper, die mit dem Schwermetall getränkt sind. Diese können ausgebildet sein als Schwamm oder als aus Faserstoff, insbesondere Papier, bestehende Streifen.

Schließlich eignet sich auch ein Behälter, dessen Innenwand mit dem antimikrobiell (oligodynamisch) wirksamen Schwermetall beschichtet ist.

Dieser Behälter läßt sich einfach herstellen und kann beispielsweise als Kunststoff-Formteil ausgebildet sein. Um eine möglichst geringe Schicht in der Größenordnung von 0,1 μm zu erzielen, wird bevorzugt das Schwermetall aufgedampft, was insbesondere nach einem bekannten Vakuumaufdampfverfahren erfolgen kann.

E 124

Als antibakteriell bzw. antimikrobiell wirksames Schwermetall sind vorteilhaft Antimon und Silber, insbesondere Feinsilber (99,9 %), wobei von den Verunreinigungen etwa 0,5 % aus Kupfer, Gold, Palladium, Aluminium und Zinn bestehen, geeignet.

Da die Pflege- und/oder Aufbewahrungslösung nur wenig oder kein Konservierungsmittel enthält, besitzt dieses Aufbewahrungsmilieu praktisch eine unbegrenzte Haltbarkeit. Eine Zersetzung von Konservierungsmitteln und deren Einwandern in die Verpackung kann nicht auftreten. Soweit das Schwermetall, insbesondere Silber, von einem in den Behälter bzw. die Lösung eingebrachten Depotträger geliefert wird, läßt sich dieser Träger in trockenem Zustand bei guter Stabilität über einen langen Zeitraum hin ohne weiteres lagern und zur Verfügung stellen.

Als Kunststoffe für den Behälter eignen sich solche, die ohne Verwendung von Zwischenschichten sich bedampfen lassen. Hier eignet sich insbesondere Polystyrol. Die Behälter können napfförmig ausgebildet sein, wobei je zwei Behälter in einem Gehäuse zur Bildung eines Aufbewahrungsetuis eingesetzt und mit einem Verschluß versehen werden können. Der als aufsetzbarer oder aufschraubbarer Deckel ausgebildete Verschluß kann einen Dichtungseinsatz mit ringförmigem Dichtungsrand am Umfang besitzen und einen mit Vorsprüngen versehenen kalottenförmigen mittleren Teil aufweisen, durch den die Linse in die konservierungsmittelfreie Pflege -und/oder Aufbewahrungslösung beim Aufschrauben des Deckels eingetaucht wird. Zur Bildung der Vorsprünge eignet sich ein sternförmiges Profil auf der in das Behälterinnere gerichteten Kalotte.

E 124

In den beiliegenden Figuren sind Ausführungsbeispiele der Erfindung dargestellt. Anhand dieser Figuren soll die Erfindung noch näher erläutert werden. Es zeigen:

| | |
|---|---|
| Fig. 1 | in perspektivischer Ansicht ein mit zwei Behältern ausgestattetes Etui; |
| Fig. 2 | eine Ansicht von unten des Verschlußdeckels; |
| Fig. 3 | eine Seitenansicht des Verschlußdeckels und |
| Fig. 4a bis d | verschiedene Ausführungsformen für die das antimikrobiell wirkende Schwermetall abgebende Einheiten. |

Beim Ausführungsbeispiel der Figur 1 bis 3 besitzt das Etui, welches zur Aufbewahrung bzw. Pflege der Kontaktlinsen dient, ein Gehäuse 1, in welchem als Behälter 2 für die Kontaktlinsen zwei napfförmige Einsätze aus Polystyrol, deren Innenwandung 3 mit Silber beschichtet ist, eingesetzt sind. Natürlich kann auch der gesamte Einsatz der Einfachheit halber mit dem antimikrobiell wirksamen Schwermetall, insbesondere Silber, beschichtet sein. Auf einem überstehenden Rand 4 kann ein Abschlußdeckel 5 aufgeschraubt werden. Der Abschlußdeckel 5, der kappenförmig ausgebildet ist, besitzt einen Einsatz 6 mit einem umlaufenden Dichtungsrand 7, der bei aufgeschraubtem Abschlußdeckel 5 auf der oberen Kante des überstehenden Randes 4 aufliegt. Ferner besitzt der Einsatz 6 einen kalottenförmigen mittleren Teil 8, der mit einem sternförmig vorstehenden Profil 9 versehen ist. Durch den kalottenförmigen Teil 8 wird die Kontaktlinse mit Sicherheit in die im Innern des Behälters befindliche Pflege- und/oder Aufbewahrungs-

E 124

lösung, die konservierungsmittelfrei ist, eingetaucht.

Auf der Unterseite des Gehäuses 1 kann ferner ein Bodenteil 11 vorgesehen sein, das abnehmbar ist und einen Spiegel auf der Unterseite des
Gehäuses freilegt, durch den ein erleichtertes Einsetzen und Entfernen der Kontaktlinsen in die und aus den Augen ermöglicht wird. Zur
Befestigung des Bodenteiles 11 dient ein einfacher Klemmechanismus
in Form von verformbaren vorstehenden Zapfen, die in den Figuren
nicht näher dargestellt sind.

Während beim Ausführungsbeispiel der Figuren 1 bis 3 als Schwermetall- bzw. Silberdepot die beschichtete Behälterinnenwand dient,
ist es auch möglich, das Schwermetalldepot auf einen Träger, der in
den unbeschichteten Behälter bzw. das Aufbewahrungsmilieu eingelegt
wird, aufzubringen. Dieser Träger kann Tablettenform besitzen
und als Preßling 10 (Fig. 4a) aus einem Schwermetallsalz, z. B. Silbersalz, oder als mit einem elementaren Schwermetall überzogener
Körper 12 (Fig. 4b) oder als poröser Körper 13 (Fig. 4c), der mit
einem Schwermetall getränkt ist, ausgebildet sein. Vorteilhaft läßt
sich auch ein mit Silber getränkter Papierstreifen 14 (Fig. 4d) verwenden.

Belastungsversuche

Beispiel 1

Es wurden 10 napfförmige Behälter aus Polystyrol, welche mit 0,1 μm
Silber beschichtet waren, Belastungstests unterzogen. Vor der Durchführung der Belastungstests wurden die Behälter durch Einlegen in
70 %iger Isopropanol 60 Minuten desinfiziert und nach Abgießen des

F 15

Alkohols in einem Exikator luftgetrocknet. Es wurden bei der Durchführung der Versuche folgende Testkeime verwendet:

Staph. aureus ATCC 6538

E. Coli ATCC 8739

Ps. aeruginosa ARCC 9027

Candida albicans ATCC 10231

Aspergillus niger ATCC 16404.

Die Bakterienstämme und der Candida albicans-Stamm stammten aus einer ca. 18 Std. alten Flüssigkultur und Asp. niger von einer ca. zwei Wochen alten Sporenplatte. Bakterien und Keime wurden in steriler phys. NaCl-Lösung aufgenommen, die Aspergillus-Sporen im selben Milieu mit einem Zusatz von 0,05 % Tween 80.

Die Zelldichte wurde mikroskopisch auf $10^5$/ml eingestellt. Mit diesen Suspensionen erfolgte der Testansatz, gleichzeitig aber auch die Bestimmung der koloniebildenden Einheiten (KBE) pro ml.

Nach Einwirkungszeiten von 1, 4, 6 und 10 Stunden bei Zimmertemperatur erfolgten Entnahmen von jeweils 0,1 ml, deren Gehalt an KBE/ml nach Vorverdünnungen auf $10^{-1}$ und $10^{-2}$ bei Benutzung eines Volumens von 0,02 ml und adäquater fester Nährmedien bestimmt wurde (für Bakterien: Tryptone Soya Medium; für die Pilze: Sabouraud-Agar).

Nachweisgrenze: 500 KBE/ml.

Die Ergebnisse sind in der anschließenden Tabelle wiedergegeben.

E 124

| Testkeim | Keime/ml i. Ansatz mikroskop. $\times 10^5$ | KBE $\times 10^5$ | Ansatz | Std. Lagerzeit – Abnahme KBE/ml in % | | | |
|---|---|---|---|---|---|---|---|
| | | | | 1 | 4 | 6 | 10 |
| Staph. aureus | 1 | 0,7 | a | 69,6 | 95,4 | 97,9 | > 99,3 |
| | | | b | 36,4 | 88,9 | 93,6 | > 99,3 |
| E. coli | 1 | 1,9 | a | 28,9 | 98,2 | > 99,7 | > 99,7 |
| | | | b | 34,2 | 98,4 | 99,5 | > 99,5 |
| Ps. aeruginosa | 1 | 6 | a | 80,0 | > 99,9 | > 99,9 | > 99,9 |
| | | | b | 71,7 | > 99,9 | > 99,9 | > 99,9 |
| Candida albicans | 1 | 0,8 | a | 25,0 | > 99,4 | > 99,4 | > 99,4 |
| | | | b | 56,3 | > 99,4 | > 99,4 | > 99,4 |
| Aspergillus niger | 1 | 0,6 | a | 58,3 | 99,2 | · 94,6 | 99,2 |
| | | | b | 33,3 | > 99,2 | > 99,2 | > 99,2 |

Die eingezeichnete Grenze zeigt die im Doppelansatz ermittelten Grenzzeiten an.

Ansatz a:   Komplette Füllung der Behälter    = 4,31 ± 0,15 ml

Ansatz b:   etwa halbe Füllung der Behälter   = 2,45 ± 0,09 ml

0049767

Die im Doppelansatz pro Test-Spezies ermittelten Resultate ergaben folgendes Bild:

Staph. aureus und E. coli waren nach einer Aufbewahrungszeit von 10 Std. in beiden Ansätzen abgetötet (unter der Nachweisgrenze). Bei Ps. aeruginosa und bei Candida albicans war dies schon nach 4 Stunden der Fall. Asp. niger war in einem Ansatz schon nach 4 Stunden nicht mehr nachweisbar, beim zweiten Ansatz aber auch noch - wenn auch in sehr geringer Menge - nach 10 Stunden.

Beispiel 2

Für die Durchführung der Belastungsversuche wurden Behälter aus Polystyrol verwendet, die nicht mit Schwermetall beschichtet waren. Für das Aufbewahrungsmilieu wurden Lösungen verwendet, deren Bestandteile innerhalb der folgenden Bereiche lagen:

Aufbewahrungsmilieu

| | |
|---|---|
| $NaH_2PO_4$ x $2H_2O$ | 0,1 - 0,3 % |
| $Na_2HPO_4$ x $2H_2O$ | 0,5 - 1,5 % |
| Borat | 0,5 - 1,5 % |
| NaCl | 0,005 - 0,015 % |

eingestellt auf pH 7,2

In 2 ml des Aufbewahrungsmilieus wurde ein Papierstreifen, der mit Silber versehen war, eingelegt, wobei je nach Zusammensetzung des Aufbewahrungsmilieus von dem als Silberdepot wirkenden Papierstreifen 0,7 - 1,5 ppm Silber an das Aufbewahrungsmilieu abgegeben wurden. Der Papierstreifen kann mehrmals verwendet werden (7mal oder

E 124

mehr ja nach Beladungsmenge). Die frei werdende Menge des Silbers hängt jeweils von der Zusammensetzung des Aufbewahrungsmilieus ab. Nach jeder Anwendung wird das Aufbewahrungsmilieu erneuert.

Bei der mikrobiologischen Prüfung wurden nach vier Stunden gute Ergebnisse erzielt. Diese sind in der folgenden Tabelle angegeben:

Mikrobiologische Ergebnisse (versilberter Papierstreifen)

| Keime | Einsaat KBE/ml $10^5$ | Abtötung nach vier Stunden |
|---|---|---|
| Staphylococcus aurus ATCC 6538 | 6,3 | 99,96 % |
| Candida albicans ATCC 10231 | 0,79 | 99,98 % |
| Aspergillus niger ATCC 16404 | 3 | 99,8 % |

Das Aufbewahrungsmedium kann in 2 ml-Packungen als Einmaldosis angeboten werden. Es sind jedoch auch 4 ml-Packungen möglich, wobei diese Gesamtmenge auf die beiden Behälter (Behälterinhalt 2 ml) eines Etuis verteilt werden können.

E 124

Patentansprüche:

1. Anordnung zur Pflege und/oder Aufbewahrung von Kontaktlinsen mit einem Behälter, in dessen Innern die Kontaktlinse in eine Pflege- und/oder Aufbewahrungslösung eingelegt wird, dadurch gekennzeichnet,

daß mit der Pflege- und/oder Aufbewahrungslösung eine ein antimikrobiell (oligodynamisch) wirksames Schwermetall in die Pflege- und/oder Aufbewahrungslösung abgebende Einheit (3, 10, 12, 13, 14) in Berührung steht.

2. Anordnung nach Anspruch 1, dadurch gekennzeichnet,

daß die Einheit (10, 12, 13, 14) als mit einem Schwermetalldepot ausgestatteter Träger ausgebildet ist.

3. Anordnung nach Anspruch 1 oder 2, dadurch gekennzeichnet,

daß die Einheit (10) als aus Schwermetallsalzen bestehender Preßling ausgebildet ist.

4. Anordnung nach Anspruch 1 oder 2, dadurch gekennzeichnet,

daß die Einheit (12) als mit dem elementaren Schwermetall überzogener Körper ausgebildet ist.

E 124

5.  Anordnung nach Anspruch 1 oder 2, dadurch gekennzeichnet,

daß die Einheit (13) als mit dem Schwermetall getränkter poröser Körper ausgebildet ist.

6.  Anordnung nach Anspruch 5, dadurch gekennzeichnet,

daß der poröse Körper (13) als Schwamm ausgebildet ist.

7.  Anordnung nach Anspruch 1 oder 2, dadurch gekennzeichnet,

daß die Einheit (14) als saugfähiger, aus einem Faserstoff bestehender Körper ausgebildet ist.

8.  Anordnung nach Anspruch 7, dadurch gekennzeichnet,

daß die Einheit als Papierstreifen (14) ausgebildet ist.

9.  Anordnung nach Anspruch 1 oder 2, dadurch gekennzeichnet,

daß die Behälterinnenwand, welche von der Pflege- und/oder Aufbewahrungslösung benetzt wird, eine Schwermetallschicht (3) aufweist.

10.  Anordnung nach Anspruch 9, dadurch gekennzeichnet,

daß die Schichtdicke des Schwermetalls 0,05 µm bis 0,5 µm beträgt.

11.  Anordnung nach Anspruch 10, dadurch gekennzeichnet,

daß die Schichtdicke des Schwermetalls 0,1 µm beträgt.

E 124

12. Anordnung nach einem der Ansprüche 9 bis 11, dadurch gekennzeichnet,

daß das Schwermetall aufgedampft ist.

13. Anordnung nach einem der Ansprüche 1 bis 12, dadurch gekennzeichnet,

daß das Schwermetall Antimon ist.

14. Anordnung nach einem der Ansprüche 1 bis 12, dadurch gekennzeichnet,

daß das Schwermetall Silber ist.

15. Anordnung nach Anspruch 14, dadurch gekennzeichnet,

daß die an die Pflege- und/oder Aufbewahrungslösung abgegebene Silbermindestkonzentration 0,7 bis 1,5 ppm beträgt.

16. Anordnung nach Anspruch 14 oder 15, dadurch gekennzeichnet,

daß die Pflege- und/oder Aufbewahrungslösung besteht aus:

| | |
|---|---|
| $NaH_2PO_4$ x $2H_2O$ | 0,1 - 0,3 % |
| $Na_2HPO_4$ x $2 H_2O$ | 0,5 - 1,5 % |
| Borat | 0,5 - 1,5 % |
| NaCl | 0,005 - 0,015 % |

eingestellt auf pH 7,2

E 124

17.   Anordnung nach einem der Ansprüche 1 bis 16, dadurch gekennzeichnet,

daß der Behälter (2) aus einem Kunststoff-Formteil gebildet ist.

18.   Anordnung nach einem der Ansprüche 1 bis 16, dadurch gekennzeichnet, daß der Kunststoff für den Behälter (2) Polystyrol ist.

19.   Anordnung nach einem der Ansprüche 1 bis 18, dadurch gekennzeichnet,

daß die Pflege- und/oder Aufbewahrungslösung eine höchstens gering konservierte Kochsalzlösung ist.

FIG.1

FIG.2

FIG.3

FIG.4

a   b   c   d